# EUROPEAN PATENT APPLICATION

(11) **EP 4 338 695 A1**
(43) Date of publication of application: **20.03.2024**
(21) Application number: 23196505.4
(22) Date of filing: 11.09.2023
(51) Int. Cl.: A61B 18/00, A61B 18/12, A61B 18/14, A61B 90/00

(54) **SYSTEM FOR COMBINED ABLATION MODALITIES**

(30) Priority: 11.09.2022 US 202263405472 P; 03.11.2022 US 202217980015; 30.08.2023 US 202318458478
(71) Applicant: BIOSENSE WEBSTER (ISRAEL) LTD., 2066717 Yokneam (IL)
(72) Inventor: SHARMA, Tushar, Irvine, 92618 (US); MAFFRE, Jennifer, Irvine, 92618 (US); GOVARI, Assaf, 2066717 Yokneam (IL); VERMA, Atul, Ontario, L3Y 2P9 (CA)
(74) Representative: Carpmaels & Ransford LLP

(57) **Abstract**

The disclosed technology includes a method of ablating tissue including positioning an electrode into contact with tissue and applying a first ablation signal to the tissue. The method can include forming a first lesion comprising a first depth with little or no first temperature change in a temperature of the tissue. The method can include applying a second ablation signal to the tissue different from the first ablation signal. Applying the second ablation signal to the tissue can include forming a second lesion comprising a second depth and generating a second temperature change in the tissue different from the first temperature change by at least 10°C. The method can include forming a combined lesion comprising the first lesion and the second lesion and comprising a combined size. The combined depth can be about 20% to about 40% greater than either of the first depth and the second size.

## Description

### RELATED APPLICATIONS

This application claims the benefit of priority under 35 U.S.C. § 119 to prior filed U.S. Provisional Patent Application No. 63/405,472, filed September 11, 2022, and is a continuation-in-part of U.S. Patent Application No. 17/980,015, filed November 3, 2022 which is a continuation of U.S. Patent Application No. 16/701,989, filed 3 December 2019, the entire contents of each of which is hereby incorporated by reference as if set forth in full herein.

### FIELD

The present invention relates to a catheter that is particularly useful for performing pulsed field ablation within or near a heart. The catheter may also be useful for mapping and/or thermal ablation using radio frequency electrical signals.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals are typically located in tissue of the atria and ventricles. Regardless of source, unwanted signals are conducted elsewhere through heart tissue where they can initiate or continue arrhythmia.

Treatment of cardiac arrhythmia can include disrupting the conducting pathway of electrical signals causing arrhythmia to cease or modify the propagation of unwanted electrical signals from one portion of the heart to another. Such procedures typically include a two-step process: (1) mapping; and (2) ablation. During mapping, a catheter having an end effector having preferably a high density of electrodes is moved across target tissue, electrical signals are acquired from each electrode, and a map is generated based on the acquired signals. During ablation, nonconducting lesions are formed at regions selected based on the map to disrupt electrical signals through those regions. Presently the most common ablation technique involves applying radio frequency ablation (RFA) electrical signals via electrodes to tissue to generate heat. Irreversible electroporation (IRE) ablation is a more recently developed technique which involves applying short duration high voltage pulses across tissue to cause cell death, sometimes referred to as pulsed field ablation (PFA). Typically, RFA and PFA are applied as separate and distinct techniques. In the context of this disclosure, RFA may be interchangeably referred to as RF and PFA may be referred to as PF.

Differing objectives of mapping, ablation with RF signals, and IRE ablation generally result in differing catheter design goals. As a non-exhaustive list, some catheters having a circular, semicircular, or helical end effector for mapping and/or ablation are described in U.S. Patent No. 6,973,339, U.S. Patent No. 7,371,232, U.S. Patent No. 8,275,440, U.S. Patent No. 8,475,450, U.S. Patent No. 8,600,472, U.S. Patent No. 8,608,735, U.S. Patent No. 9,050,010, U.S. Patent No. 9,788,893, U.S. Patent No. 9,848,948, and U.S. Patent Pub. Nos. 2017/0100188, and 2021/0369338, U.S. Patent Application No. 17/570,829 (published as 2023/0009573), and U.S. Provisional Patent Nos. 63/336,094, and 63/220,312 each of which are incorporated herein by reference with a copy provided in the Appendix included in priority application no. 63/405,472.

### SUMMARY

Generally, examples presented herein can include a method of ablating tissue. The method can include positioning an electrode into contact with the tissue and applying, with the electrode, a first ablation signal to the tissue. Applying the first ablation signal to the tissue can include forming a first lesion comprising a first depth with little or no first temperature change in a temperature of the tissue.

The method can include applying, with the electrode, a second ablation signal to the tissue different from the first ablation signal. Applying the second ablation signal to the tissue can include forming a second lesion comprising a second depth and generating a second temperature change in the tissue different from the first temperature change by at least 10°C.

The method can include forming a combined lesion comprising the first lesion and the second lesion and comprising a combined size. The combined depth can be about 20% to about 40% greater than either of the first depth and the second size.

The first ablation signal and the second ablation signal can be applied to the tissue at least one of sequentially or simultaneously.

The positioning step can include applying a contact force between the tissue and the electrode of about 5 grams to about 40 grams.

The first ablation signal can be one of a radio frequency (RF) signal or a pulsed field (PF) signal, and the second ablation signal is the other of the RF signal or the PF signal.

The method can further include setting a power of the RF signal to about 1 Watt to about 400 Watt, maintaining the RF signal for about 1 second to about 60 seconds, and generating one of the first or second temperature change to about 20°C to about 70°C.

The method can further include setting a voltage of the PF signal to about 900 volts to about 3000 volts.

The RF signal can form one of the first depth or the second depth between about 3mm to about 5 mm.

The PF signal can form one of the first depth or the second depth between about 4mm to about 6mm.

The disclosed technology can include a system for electrophysiology uses. The system can include an alternating current (AC) signal generator configured to provide radiofrequency signals at high power, a direct current (DC) signal generator configured to provide high voltage pulses, and a catheter having an end effector.

The end effector can be electrically coupled to the AC signal generator and the DC signal generator. The end effector can include at least one electrode disposed on the end effector so that the at least one electrode delivers the high voltage pulses from the at least one electrode to organ tissue inside a patient to first and second return electrodes coupled to the outside body of the patient and delivers the radiofrequency signal between the at least one electrode to one of the first or second return electrodes.

The radiofrequency signals and the high voltage pulses can be applied either sequentially or simultaneously to the organ tissue.

The end effector can include a cylindrical member with a distal tip electrode and irrigation ports disposed on the cylindrical member to provide irrigation fluid proximate the distal tip electrode.

The distal tip electrode can be coupled to a force sensor. The radiofrequency signals can be applied with a contact force of approximately 5 grams or more. The radiofrequency signals can be provided of at least 25 Watts of power at approximately 350kHZ to approximately 500 kHz. The radiofrequency signals can include a frequency from 350kHZ to about 500 kHZ and the radiofrequency signals can be provided for a duration of at least 1 second.

The high voltage pulses can include an amplitude of at least 800 V. A duration of each of the high voltage pulse can be less than 20 microseconds. A plurality of high voltage pulses can provide a pulse train of approximately 100 microseconds. A time gap of any value selected from 0.3 to 1000 milliseconds can be provided between adjacent pulse trains. A plurality of pulse trains can provide a PFA burst. The PFA burst can include any value from 2 to 100 pulse trains with a duration of the PFA burst comprising any value selected from zero to 500 milliseconds. The high voltage pulse can provide approximately 60 Joules or less.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, systems, and methods by way of example only, not by way of limitation.
FIG. 1 is a schematic pictorial illustration of a medical system including an example medical probe which can be used in accordance with the disclosed technology;
FIG. 2A is a side view of a catheter for use with the system of Fig. 1, in accordance with the disclosed technology;
FIG. 2B is a perspective view of a catheter for use with the system of Fig. 1, in accordance with the disclosed technology;
FIG. 3 is a schematic sectional view of a heart chamber with a catheter in contact with the heart wall inside the chamber, in accordance with the disclosed technology;
FIGs. 4A and 4B are pictures depicting the depth of tissue treated using RF energy and PFA energy, in accordance with the disclosed technology;

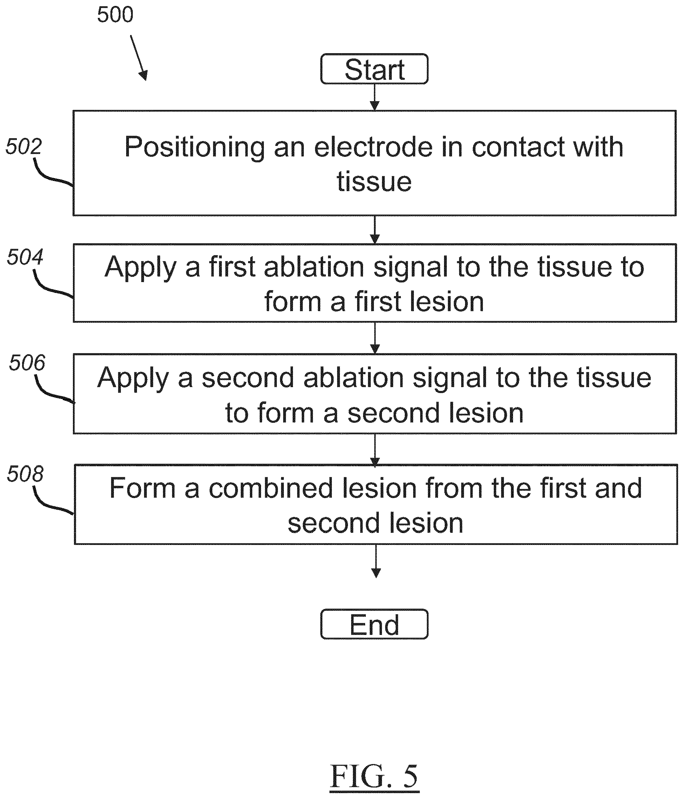

FIG. 5 illustrates a flowchart of a method of ablating tissue using RF energy and PFA energy, in accordance with the disclosed technology;
FIG. 6A illustrates a basket catheter, in accordance with the disclosed technology;
FIG. 6B illustrates a circular catheter, in accordance with the disclosed technology;
FIG. 6C illustrates a planar array catheter, in accordance with the disclosed technology.

### DETAILED DESCRIPTION

Documents incorporated by reference herein are to be considered an integral part of the application except that, to the extent that any terms are defined in these incorporated documents in a manner that conflicts with definitions made explicitly or implicitly in the present specification, only the definitions in the present specification should be considered.

Although example embodiments of the disclosed technology are explained in detail herein, it is to be understood that other embodiments are contemplated. Accordingly, it is not intended that the disclosed technology be limited in its scope to the details of construction and arrangement of components set forth in the following description or illustrated in the drawings. The disclosed technology is capable of other embodiments and of being practiced or carried out in various ways. Features of embodiments disclosed herein, including those disclosed in the attached Appendix, can be combined as understood by a person skilled in the pertinent art according to the teachings herein.

As used herein, the terms "about" or "approximately" for any numerical values or ranges indicate a suitable dimensional tolerance that allows the part or collection of components to function for its intended purpose as described herein. More specifically, "about" or "approximately" can refer to the range of values ±20% of the recited value, e.g. "about 90%" can refer to the range of values from 71% to 99%.

As discussed herein, the term "ablate" or "ablation", as it relates to the devices and corresponding systems of this disclosure, refers to components and structural features configured to reduce or prevent the generation of erratic cardiac signals. Non-thermal ablation includes use of irreversible electroporation (IRE) to cause cell death, referred throughout this disclosure interchangeably as pulsed electric field (PEF) and pulsed field ablation (PFA). Thermal ablation includes use of extreme temperature to cause cell death and includes RF ablation. Ablating or ablation as it relates to the devices and corresponding systems of this disclosure is used throughout this disclosure in reference to ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" as it generally relates to known methods, devices, and systems includes various forms of bodily tissue ablation as understood by a person skilled in the pertinent art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned internally inside the body at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site, typically outside the body via contact patches.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

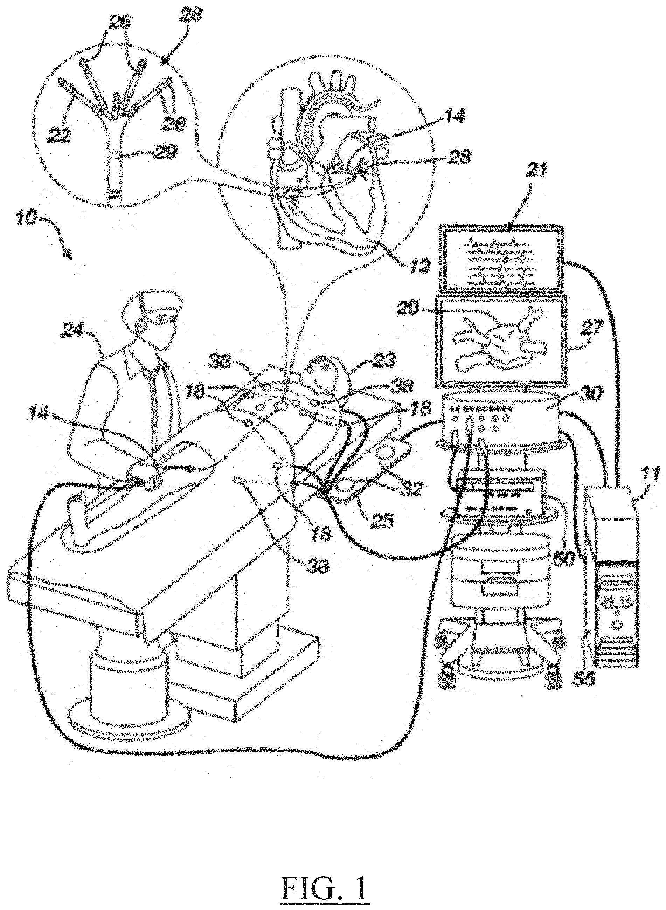

Reference is made to FIG. 1 showing an example catheter-based electrophysiology mapping and ablation system 10. System 10 includes multiple catheters, which are percutaneously inserted by physician 24 through the patient's vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip 28 of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over a plurality of spines 22 at distal tip 28 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182. Electrode patch 38 (one or more) can be used as a return electrode (also called an indifferent electrode) during a unipolar mode of ablation whereby the electrical energy is provided by the electrode(s) on the catheter so that the electrical energy completes a circuit via the tissues to the indifferent (or return) electrode(s) 38. In practice, where the electrode patches 38 are not sufficiently large enough in surface area to handle the power delivery, two separate return electrode patches (with surface areas larger than patches 38) are used separately from the electrode patch 38.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

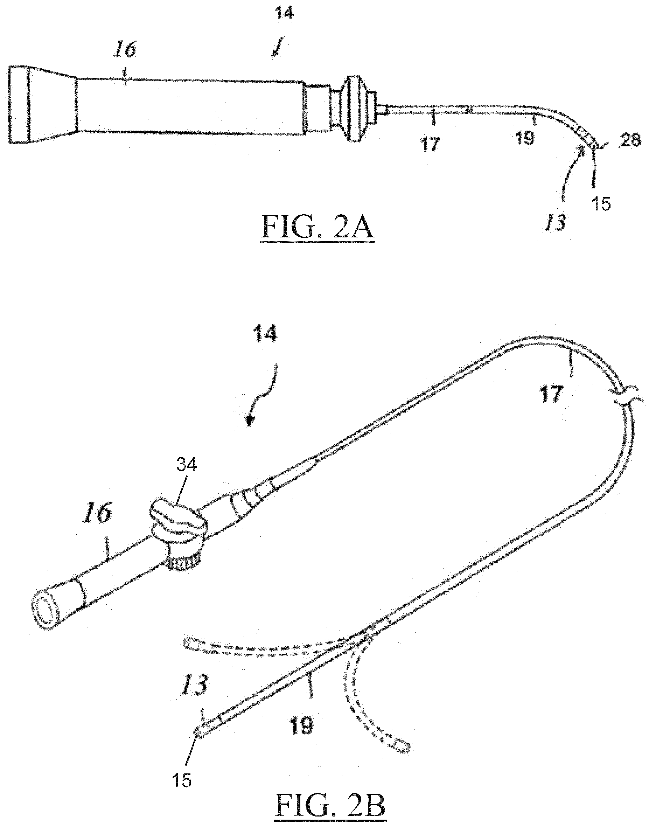

As shown in FIG. 2A, the catheter 14 can include an elongated catheter body 17, a deflectable intermediate section 19, a distal section 13 carrying at least a tip electrode 15 on its distal tip end 28, and a control handle 16. The catheter 14 can be one that is a steerable multi-electrode luminal catheter with a deflectable tip designed to facilitate electrophysiological mapping of the heart 12 and to transmit radiofrequency (RF) and pulse field (PF) current to the tip electrode 15 for ablation purposes. A physician 24, such as a cardiologist, can insert catheter 14 through the vascular system of a patient 23 so that a distal section 13 of the catheter enters a chamber of the patient's heart 12. The physician 24 advances the catheter so that a distal tip 28 of the catheter engages endocardial tissue at a desired location or locations. Catheter 14 is connected by a suitable connector at its proximal end to workstation 55. The workstation 55 may include the ablation energy generator 50, which supplies high-frequency electrical energy (AC or DC) via the catheter 14 for ablating tissue in the heart 12 at the locations engaged by the distal section 13. For ablation, the catheter 14 can be used in conjunction with a dispersive pad (e.g., return electrode or indifferent electrode). In this respect, the catheter 14 can include a shaft that measures 7.5 F with 8 F ring electrodes. The catheter 14 can also have a force-sensing system near the distal tip electrode 15 that provides a real-time measurement of contact force (as well as the angle of contact between tip electrode 15 and tissue) between the catheter tip 15 and the heart 12 wall.

As shown in FIG. 2B, distal tip section 13 can include an electrode assembly 15 and at least one micro-element having an atraumatic distal end adapted for direct contact with target tissue. Catheter body 17 can have a longitudinal axis, and an intermediate section 19 distal of the catheter body 17 that can be uni- or bi-directionally deflectable off-axis from the catheter body 17. Distal of the intermediate section 19 is the electrode assembly carrying at least one electrode 15. Proximal of the catheter body is control handle 16 that allows an operator to maneuver the catheter, including deflection of the intermediate section 19.

The elongated catheter body can be a relatively high torqueable shaft with the distal tip section 13 attached to the deflectable intermediate section 19 and containing an electrode assembly 15 with an array of electrodes. For example, the distal tip section 13 can include a 3.5 mm tip dome with three microelectrodes. All of the electrodes may be used for recording and stimulation purposes. A rocker lever 34 can be used to deflect the distal tip section 13. The high-torque shaft also allows the plane of the curved tip to be rotated to facilitate accurate positioning of the catheter tip at the desired site. Three curve types configurations designated "D," "F," and "J" are available. The electrode assembly 15 serves to deliver ablative energy from the ablation generator 50 to the desired ablation site. The electrode assembly 15 and ring electrodes can be made from noble metals. In some examples, the catheter 14 can also include six thermocouple temperature sensors that are embedded in the 3.5 mm tip electrode 15.

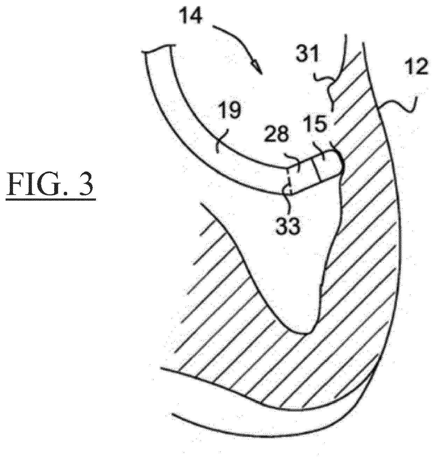

FIG. 3 is a schematic sectional view of a chamber of a heart 12, showing a flexible deflectable intermediate section 19 of a catheter 14 inside the heart. The catheter 14 is typically inserted into the heart percutaneously through a blood vessel, such as the vena cava or the aorta and then inserted via the septum to arrive at the endocardial heart chamber. The electrode 15 on a distal tip 28 of the catheter engages endocardial tissue 31. Pressure exerted by the distal tip against the endocardium deforms the endocardial tissue locally, so that electrode 15 contacts the tissue over a relatively large area. In the pictured example, the electrode 15 engages the endocardium at an angle, rather than head-on. Distal tip 28 therefore bends at an elastic joint 33 relative to deflectable intermediate section 19 of the catheter. The bend facilitates optimal contact between the electrode and the endocardial tissue.

Because of the elastic quality of joint 33, the angle of bending of the joint is typically proportional to the pressure exerted by tissue 31 on distal tip 28 (or equivalently, the pressure exerted by the distal tip on the tissue). Measurement of the bend angle thus gives an indication of this pressure. The pressure indication may be used by the operator of catheter 14 to ensure that the distal tip is pressing against the endocardium firmly enough to give the desired therapeutic or diagnostic result, but not so hard as to cause undesired tissue damage. US Patent Nos. 8,357,152, 9,492,639 and 10,688,278 whose disclosures are incorporated herein by reference, describes a system that uses a pressure-sensing catheter in this manner. Catheter 14 may be used in such a system.

Ablation energy generator 50 can generate radio frequency (RF) current pursuant to known RF generators as disclosed in US Patent No. 5,906,614 and US Patent No. 10,869,713 which discloses high power RF generators, both disclosures are incorporated herein by reference. RF current creates ablation lesions by a thermal process. RF ablation raises tissue temperature and destroys cells through heating. Further, ablation energy generator 50 can also generate pulse field (PF) current to create lesions using irreversible electroporation (IRE). IRE is a predominantly non-thermal process that destroys cells by disrupting the cell membranes. Discussions of a dual mode ablation energy generator 50 capable of producing both RF and PF signals can be found in US Publication No. 2021/0161592. US Publication No. 2021/0161592 further discusses using PF and RF ablation in combination and is incorporated herein by reference with a copy provided in the Appendix included in priority application no. 63/405,472.

Using the above ablation energy generator 50 and the end effectors discussed above and below, can be used in method for increased lesion formation using RF and PF signals. These lesions are formed in the patient's tissue and in one example, for pulmonary vein isolation techniques.

Typical RF ablation creates lesions with a depth into the tissue of about 3mm to about 5 mm. One example of the parameters used to create the RF ablated lesions are setting a power of the RF signal to about 1 Watt to about 400 Watts. Further, the RF signal is maintained for about 1 second to about 60 seconds.

Given that RF ablation uses heat to damage the tissue, RF signals typically generate a temperature change in the tissue from about 20°C to about 70°C above body temperature.

Typical PF is different from RF ablation in that at least the PF signals generate a temperature change of only a few degrees. PF typically creates lesions in the patient's tissue sized between about 4mm to about 6mm. To create the lesions, a voltage of the PF signal is set to about 800 volts to about 3000 volts. Further, the PF signals are typically generated using particular waveforms.

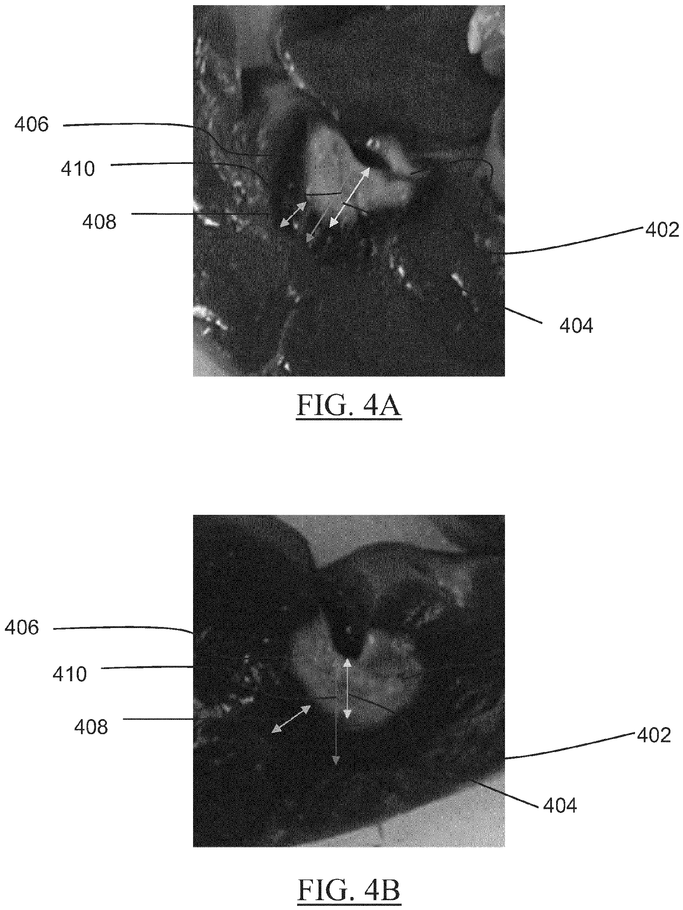

However, one aspect of this invention uses both RF and PF signals on the same portion of tissue to create larger, i.e., deeper lesions than either RF signals or PF signals can create alone. FIGs. 4A and 4B illustrate tissue treated with an example of the invention. The pale/white tissue is a RF ablation lesion 402. In this example, the RF ablation lesion was formed by generating a RF signal at about 50W of power and for about 10 seconds of duration. The RF ablation signal formed a lesion with a particular depth 404. Then a PF signal was applied to the same location on the tissue. The PF signal caused a PF lesion 406. This is illustrated by the darker tissue surrounding the RF ablation lesion 402. The PF signal was generated using standard PF protocols, as described in US Patent Publication No. 2021/0161592.

Applying the PF signal to the same location as where the RF signal was applied results in a combined lesion. The combined lesion depth 410 is formed from the RF ablation lesion 402 and the PF ablation lesion 406. The combined lesion's depth or depth 410 is larger or deeper than either the RF ablation lesion depth 404 or the PF lesion depth 408. The combined lesion depth 410 can be about 20% to about 40% greater than the RF ablation lesion depth 404 and the PF ablation lesion depth 408. One consideration for this surprising result is that RF signal "primes" or "prepares" the non-ablated tissue surrounding the RF ablated region 402 so that the PF signal can reach deeper into the tissues. This priming can weaken the cells in that area below region 402 so the PF ablation signal can create a deeper lesion 408. Conversely, application of the PF signal first can disrupt the voltage differential in cell walls so that when RF signal is applied, the lesion can be just as deep. Both the PF signal and the RF signal can also be applied simultaneously to substantially achieve the same lesion depth. It should be noted here that lesion "depth" (maximum extent measured from electrode contact point to deep inside a sectioned tissue) is measured to the maximum depth for ease of measurement but that an average depth can also be utilized to quantify the ablative effect of each ablation modality.

We have heretofore devised an ablation system for electrophysiology ablation that provides a combined lesion depth via two distinct energy modalities: (a) an alternating current (AC) signal generator configured to provide radiofrequency signals at high power and (b) a direct current (DC) signal generator configured to provide high voltage pulses. The system also includes a catheter having an end effector electrically coupled to the AC signal generator and the DC signal generator. The end effector can have at least one electrode disposed on the end effector so that the electrode delivers the high voltage pulses from the at least one electrode to organ tissue inside a patient to first and second return electrodes coupled to the outside body of the patient and deliver the RF signal between the at least one electrode to one of the first or second return electrodes. The RF signals and the high voltage pulses can be applied either sequentially or simultaneously to the organ tissue.

In an example, the end effector can have a cylindrical member with a distal tip electrode and irrigation ports disposed on the cylindrical member to provide irrigation fluid proximate the distal tip electrode.

Another example can have the distal tip electrode coupled to a force sensor. Further, the radiofrequency signals can be applied with a contact force of approximately 5 grams or more. Also, the radiofrequency signals can be provided having at least 25 Watts of power (up to 400 Watts) at approximately 350kHZ to approximately 500 kHz. The radiofrequency signals can also include a frequency from 350kHZ to about 500 kHZ and the radiofrequency signals can be provided for a duration of at least 1 second to about 240 seconds.

For other examples, the high voltage pulses can include an amplitude of at least 800 V to about 3000V. In addition, a duration of each of the high voltage pulse can be less than 20 microseconds and provide a pulse train of approximately 100 microseconds. A time gap of any value selected from 0.3 to 1000 milliseconds can be provided between adjacent pulse trains. These pulse trains can provide a PF burst. The PF burst can have any value from 2 to 100 pulse trains with a duration of the PF burst comprising any value selected from zero to 500 milliseconds. Furthermore, the high voltage pulse can provide approximately 60 Joules or less.

By virtue of the system and disclosure provided herein, we have devised a method 500 of ablating tissue using the present invention, as indicated in Fig. 7. The method 500 can include positioning an electrode into contact with the tissue (Step 502). Once in contact, the method 500 can include applying, with the electrode, a first ablation signal to the tissue (Step 504). In one example, the first ablation signal can be the RF ablation signal. However, in other examples, the PF ablation signal can be the first ablation signal. The first ablation signal can form a first lesion comprising a first depth and generate a first temperature change in the tissue.

A second ablation signal can be applied to the tissue with the electrode to form a second lesion in the tissue (Step 506). In the example, the second ablation signal can be different from the first ablation signal. The second lesion can be formed with a second size. The second ablation signal can generate a second temperature change in the tissue different from the first temperature change by at least 10°C. As above, if the RF signal is the first signal, the PF signal can be the second signal and when the PF signal is the first signal the RF signal can be the second signal. Where RF ablation causes tissue temperature changes greater than 20°C, the PF causes a temperature change of just a few degrees.

Alternately a first lesion can be formed with little or no first temperature change in a temperature of the tissue and a second lesion can be formed by generating a second temperature change in the tissue different from the first temperature change by at least 10°C.

The method can include 500 forming a combined lesion (Step 508) which can result from applying the first ablation signal and the second ablation signal. The deeper/larger combined lesion can be formed from the combination of the first lesion and the second lesion having a combined size. The combined depth can be about 20% to about 40% greater than either of the first depth and the second size. Further, the method 500 can include applying the first ablation signal and the second ablation signal sequentially. This can include first applying the RF signal and then applying the PF signal or visa-a-versa. However, given that the RF signals can be generated using alternating current (AC) and the PF signals can be generated using direct current (DC), another example can have both signals generated at the same time or at least having some overlap of application of the RF and PF signals. Additionally, contact force between the tissue and the electrode is known to be a factor in the effectiveness of creating a lesion. In one example the contact force can be about 5 grams to about 40 grams.

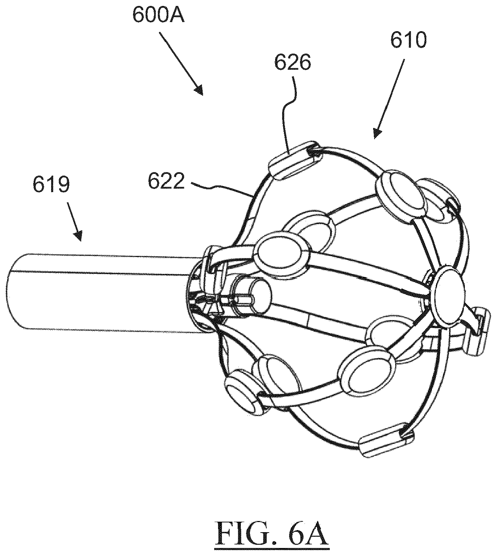

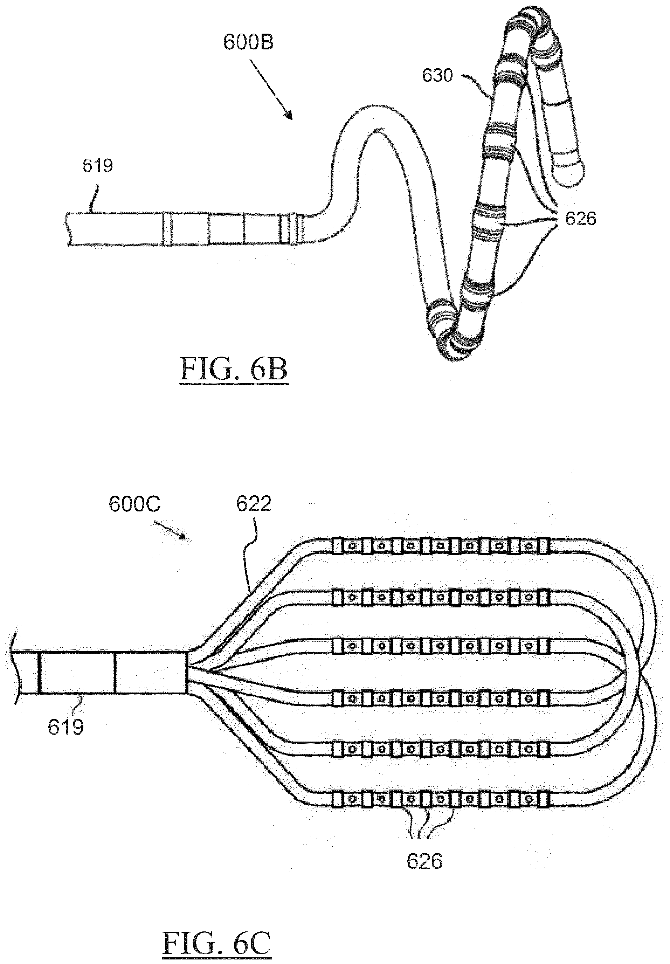

Although the present disclosure is described as being applicable to the catheter 14 having a tip electrode 15 as shown and described in relation to FIGs. 2A-3, the disclosed technology is not so limited and can be applicable to catheters having other configurations. For example, the disclosed technology can be applicable to basket catheters 600A (as shown in FIG. 6A and described in U.S. Provisional Patent Application No. 63/336,094, incorporated herein by reference), circular catheters 600B having a circular region generally transverse to a longitudinal axis of the catheter (as shown in FIG. 6B and described in U.S. Provisional Patent Application No. 63/220,312, incorporated herein by reference), and planar array catheters 600C having a planar array of electrodes (as shown in FIG. 6C and described in U.S. Patent Publication No. 2021/0369338). Furthermore, the disclosed technology can be applicable to catheters with or without the ability to deliver irrigation to an area proximate the electrode. Further still, the disclosed technology can be applicable to catheters having a force sensor configured to detect a force applied to a distal end of the catheter as well as catheters that do not have a force sensor. In other words, the disclosed technology can be applicable to a broad range of catheters that have electrodes configured to ablate tissue.

FIG. 6A is a perspective view of a basket catheter 600A having basket electrode assembly 610 attached to a distal end of a deflectable intermediate section 619. The basket electrode assembly 610 can include a plurality of spines 622 each having one or more electrodes 626 attached thereto. The electrodes 626 can be configured for mapping and/or ablation. For example, the electrodes 626 can be configured to deliver RF energy or PF energy to ablate tissue in accordance with the technology described herein. As will be appreciated, by using a basket electrode assembly 610 to deliver RF energy or PF energy, the disclosed technology can be configured to ablate a larger area of tissue than the tip electrode 15 described in relation to FIGs. 2A-3. In some examples, selected electrodes 626 on the basket electrode assembly 610 can be energized to ablate selected areas of the tissue in which the basket electrode assembly 610 is in contact with, enabling the physician 24 greater control of the basket electrode assembly 610.

FIG. 6B is an illustration of a profile view of a circular catheter 600B having a circular region 630 attached to the deflectable intermediate section 619. The circular region 630 is disposed generally orthogonal to the longitudinal axis passing through the distal end of the deflectable intermediate section 619. The circular region 630 is preferably generally perpendicular to the catheter body and forms flat circle or can be slightly helical, as shown in FIG. 6B.

The circular region 630 includes electrodes 626 distributed around its circumference. The electrodes 626 can be configured for mapping and/or ablation. During an example treatment in which the electrodes 626 are used to ablate tissue, the electrodes 626 can be configured to deliver both RF energy and PF energy to ablate the tissue in accordance with the technology disclosed herein. The depth of the generally circular region 630 can permit ablation of tissue along a diameter of a pulmonary vein or other tubular structure of or near the heart 12 when the circular main region has a diameter generally corresponding to that of a pulmonary vein or the coronary sinus. The circular arrangement of the electrodes 626 facilitates formation of a circular, or ring-shaped lesion to interrupt electrical activity through the circumference of the tubular bodily structure, thereby electrically isolating the tubular structure from tissue on the opposite side of the ring-shaped lesion. In other examples, the electrodes 626 can each be individually energized to provide RF or PF ablation only at selected portions of the tissue.

FIG. 6C illustrates a planar array catheter 600C having a plurality of spines 622 arranged in a plane and attached to a distal end of the deflectable intermediate section 619. Each spine 622 has one or more electrodes 626 disposed thereon. Each electrode 626 can be configured for mapping and/or ablation. For example, the electrodes 626 can be configured to deliver RF energy or PF energy to ablate tissue in accordance with the technology described herein. By arranging the electrodes 626 along the spines 622 in a planar array, the planar array catheter 600C can be configured to facilitate ablation of a greater area of tissue than the tip electrode 15 described in relation to FIGs. 2A-3. In some examples, selected electrodes 626 on the planar array catheter 600C can be energized to ablate selected areas of the tissue in which the electrodes 626 are in contact with, enabling the physician 24 greater control of the planar array catheter 600C.

As will be appreciated, the method 500 described herein can be varied in accordance with the various elements and examples described herein. That is, methods in accordance with the disclosed technology can include all or some of the steps described above and/or can include additional steps not expressly disclosed above. Certain steps can be performed in sequence or simultaneously. Further, methods in accordance with the disclosed technology can include some, but not all, of a particular step described above. Further still, various methods described herein can be combined in full or in part.

The disclosed technology can be further understood by the following clauses:
Clause 1: An ablation system for electrophysiology uses, the system comprising: an alternating current (AC) signal generator configured to provide radiofrequency signals at high power; a direct current (DC) signal generator configured to provide high voltage pulses; and a catheter having an end effector electrically coupled to the AC signal generator and the DC signal generator, the end effector comprising at least one electrode disposed on the end effector so that the at least one electrode delivers the high voltage pulses from the at least one electrode to organ tissue inside a patient to first and second return electrodes coupled to the body of the patient and deliver the radiofrequency signal between the at least one electrode to one of the first or second return electrodes.
Clause 2: The ablation system of clause 1, in which the radiofrequency signals and the high voltage pulses are applied either sequentially or simultaneously to the organ tissue.
Clause 3: The ablation system of any of the previous clauses, in which the end effector comprises a cylindrical member with a distal tip electrode and irrigation ports disposed on the cylindrical member to provide irrigation fluid proximate the distal tip electrode.
Clause 4: The ablation system of any of the previous clauses, in which the distal tip electrode is coupled to a force sensor.
Clause 5: The ablation system of any of the previous clauses, in which the radiofrequency signals are applied with a contact force of approximately 5 grams or more.
Clause 6: The ablation system of any of the previous clauses, in which the radiofrequency signals are provided with at least 25 Watts of power.
Clause 7: The ablation system of any of the previous clauses, in which the radiofrequency signals include a frequency from 350kHZ to about 500 kHZ and the radiofrequency signals are provided for a duration of at least 1 second.
Clause 8: The ablation system of any of the previous clauses, in which the high voltage pulses include an amplitude of at least 800 V.
Clause 9: The ablation system of any of the previous clauses, in which a duration of each of the high voltage pulse is less than 20 microseconds.
Clause 10: The ablation system of any of the previous clauses, in which a plurality of high voltage pulses provides a pulse train of approximately 100 microseconds.
Clause 11: The ablation system of any of the previous clauses, in which a time gap of any value selected from 0.3 to 1000 milliseconds is provided between adjacent pulse trains.
Clause 12: The ablation system of any of the previous clauses, in which a plurality of pulse trains provides a PF burst.
Clause 13: The ablation system of any of the previous clauses, in which the PF burst comprises any value from 2 to 100 pulse trains with a duration of the PF burst comprising any value selected from zero to 500 milliseconds.
Clause 14: The ablation system of any of the previous clauses, in which the high voltage pulse provides approximately 60 Joules or less.
Clause 15: The ablation system of any of the previous clauses, in which the at least one electrode is configured to deliver the high voltage pulses and the radiofrequency signal to tissue to form a combined lesion in the tissue such that the combined lesion is at least 20% greater than a lesion formed by the same radiofrequency signal alone or the same high voltage pulses alone.
Clause 16: A method of ablating tissue, comprising: positioning an electrode into contact with the tissue; applying, with the electrode, a first ablation signal to the tissue, comprising: forming a first lesion comprising a first depth with little or no first temperature change in a temperature of the tissue; and applying, with the electrode, a second ablation signal to the tissue different from the first ablation signal, comprising: forming a second lesion comprising a second size; and generating a second temperature change in the tissue different from the first temperature change by at least 10°C; and forming a combined lesion comprising the first lesion and the second lesion and comprising a combined size, the combined depth being about 20% to about 40% greater than either of the first depth and the second size.
Clause 17: The method of clause 16, wherein the first ablation signal and the second ablation signal are applied to the tissue at least one of sequentially or simultaneously.
Clause 18: The method of clause 16, wherein the positioning step comprises applying a contact force between the tissue and the electrode of about 5 grams to about 40 grams.
Clause 19: The method of clause 16, wherein the first ablation signal is one of a radio frequency (RF) signal or a pulsed field (PF) signal, and the second ablation signal is the other of the RF signal or the PF signal.
Clause 20: The method of any of clauses 16-19, further comprising: setting a power of the RF signal to about 1 Watt to about 400 Watt; maintaining the RF signal for about 1 second to about 60 seconds; and generating one of the first or second temperature change to about 20°C to about 70°C.
Clause 21: The method of clauses 19-20, further comprising: setting a voltage of the PF signal to about 900 volts to about 3000 volts.
Clause 22: The method of any of clauses 16-19, wherein the RF signal forms one of the first depth or the second depth between about 3mm to about 5 mm.
Clause 23: The method of any of clauses 16-19, wherein the PF signal forms one of the first depth or the second depth between about 4mm to about 6mm.

While the present disclosure has been described in connection with a plurality of exemplary aspects, as illustrated in the various figures and discussed above, it is understood that other similar aspects can be used, or modifications and additions can be made to the described aspects for performing the same function of the present disclosure without deviating therefrom. For example, in various aspects of the disclosure, methods and compositions were described according to aspects of the presently disclosed subject matter. But other equivalent methods or compositions to these described aspects are also contemplated by the teachings herein. Therefore, the present disclosure should not be limited to any single aspect, but rather construed in breadth and scope in accordance with the appended claims.

Aspects of the invention:
1. A method of ablating tissue, comprising:
   positioning an electrode into contact with the tissue;
   applying, with the electrode, a first ablation signal to the tissue, comprising:
      forming a first lesion comprising a first depth and a first size with little or no first temperature change in a temperature of the tissue; and
   applying, with the electrode, a second ablation signal to the tissue different from the first ablation signal, comprising:
      forming a second lesion comprising a second depth and a second size; and
      generating a second temperature change in the tissue different from the first temperature change by at least 10°C; and
   forming a combined lesion comprising the first lesion and the second lesion and comprising a combined size and a combined depth,
   the combined depth being about 20% to about 40% greater than either of the first depth and the second depth.
2. The method of aspect 1, wherein the first ablation signal and the second ablation signal are applied to the tissue at least one of sequentially or simultaneously.
3. The method of aspect 1, wherein the first ablation signal is one of a radio frequency (RF) signal or a pulsed field (PF) signal, and the second ablation signal is the other of the RF signal or the PF signal.
4. The method of aspect 3, further comprising:
   setting a power of the RF signal to about 1 Watt to about 400 Watts;
   maintaining the RF signal for about 1 second to about 60 seconds; and
   generating one of the first or second temperature change to about 20°C to about 70°C.
5. The method of aspect 4, wherein the RF signal forms one of the first depth or the second depth between about 3mm to about 5 mm.
6. The method of aspect 4, wherein the PF signal forms one of the first depth or the second depth between about 4mm to about 6mm.

## Claims

1. An ablation system for electrophysiology uses, the ablation system comprising:
an alternating current (AC) signal generator configured to provide radiofrequency signals at high power;
a direct current (DC) signal generator configured to provide high voltage pulses; and
a catheter having an end effector electrically coupled to the AC signal generator and the DC signal generator, the end effector comprising at least one electrode disposed on the end effector so that the at least one electrode delivers the high voltage pulses from the at least one electrode to organ tissue inside a body of a patient to first and second return electrodes coupled to the body of the patient and deliver the radiofrequency signal between the at least one electrode to one of the first or second return electrodes.

2. The ablation system of claim 1, in which the radiofrequency signals and the high voltage pulses are applied either sequentially or simultaneously to the organ tissue.

3. The ablation system of claim 1 or claim 2, in which the end effector comprises a cylindrical member with a distal tip electrode and irrigation ports disposed on the cylindrical member to provide irrigation fluid proximate the distal tip electrode, the distal tip electrode being coupled to a force sensor.

4. The ablation system of any preceding claim , in which the radiofrequency signals are applied with a contact force of approximately 5 grams or more.

5. The ablation system of any preceding claim, in which the radiofrequency signals are provided with at least 25 Watts of power.

6. The ablation system of any preceding claim, in which the radiofrequency signals include a frequency from 350kHZ to about 500 kHZ and the radiofrequency signals are provided for a duration of at least 1 second.

7. The ablation system of any preceding claim, in which the high voltage pulses include an amplitude of at least 800 V.

8. The ablation system of claim 7, in which the high voltage pulse provides approximately 60 Joules or less.

9. The ablation system of claim 7 or claim 8, in which a duration of each of the high voltage pulse is less than 20 microseconds.

10. The ablation system of any of claims 7 to 9, in which a plurality of high voltage pulses provides a pulse train of approximately 100 microseconds.

11. The ablation system of claim 10, in which a time gap of any value selected from 0.3 to 1000 milliseconds is provided between adjacent pulse trains.

12. The ablation system of claim 10 or claim 11, in which a plurality of pulse trains provides a PF burst.

13. The ablation system of claim 12, in which the PF burst comprises any value from 2 to 100 pulse trains with a duration of the PF burst comprising any value selected from zero to 500 milliseconds.

14. The ablation system of any preceding claim, in which the at least one electrode is configured to deliver the high voltage pulses and the radiofrequency signal to tissue to form a combined lesion in the tissue such that the combined lesion is at least 20% greater than a lesion formed by the same radiofrequency signal alone or the same high voltage pulses alone.
